(19) Europäisches Patentamt
European Patent Office
Office européen des brevets

(11) EP 4 491 658 A1

(12) EUROPEAN PATENT APPLICATION
published in accordance with Art. 153(4) EPC

(43) Date of publication:
**15.01.2025 Bulletin 2025/03**

(21) Application number: **23767138.3**

(22) Date of filing: **07.03.2023**

(51) International Patent Classification (IPC):
***C08G 83/00*** (2006.01) ***C08J 9/40*** (2006.01)
***C08L 5/08*** (2006.01) ***C08L 101/00*** (2006.01)
***A61L 24/08*** (2006.01) ***A61L 24/00*** (2006.01)
***A61K 9/00*** (2006.01) ***A61K 47/30*** (2006.01)

(52) Cooperative Patent Classification (CPC):
**A61K 9/00; A61K 47/30; A61L 24/00; A61L 24/08; C08G 83/00; C08J 9/40; C08L 5/08; C08L 101/00**

(86) International application number:
**PCT/KR2023/003119**

(87) International publication number:
**WO 2023/172037 (14.09.2023 Gazette 2023/37)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **11.03.2022 KR 20220030576**
**11.03.2022 KR 20220030577**

(71) Applicant: **Genewel Co., Ltd**
**Seongnam-si, Gyeonggi-do 13211 (KR)**

(72) Inventors:
- **LEE, Hye Ri**
  **Suwon-si, Gyeonggi-do 16627 (KR)**
- **CHO, Mi Ran**
  **Yongin-si, Gyeonggi-do 17066 (KR)**
- **LEE, Seul Gi**
  **Anyang-si, Gyeonggi-do 13922 (KR)**

(74) Representative: **Dehns**
**10 Old Bailey**
**London EC4M 7NG (GB)**

# (54) PASTE COMPOSITION, BIODEGRADABLE INJECTABLE PASTE, AND METHOD FOR PRODUCING SAME

(57) The present invention relates to a paste composition, a biodegradable injectable paste, and a method for producing the same. According to the present invention, the paste composition may offer the convenience of being applied to the application site using one syringe without the need to mix using two syringes immediately before use, by providing a crosslinked hydrogel as a paste phase, as well as biocompatibility, tackiness, and the balance between viscosity and elasticity, and thus is suitable for use in applications, including hemostatic agents, bone fillers, wound dressings, drug carriers, tissue repair materials, skin soothing agents, moisturizing agents, anti-inflammatory agents, and anti-atopic agents.

FIG. 1

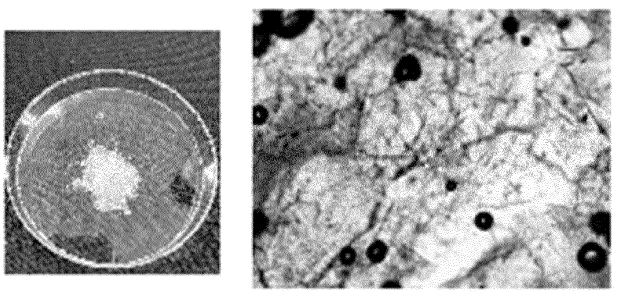



Processed by Luminess, 75001 PARIS (FR)

## Description

### Technical Field

The present invention relates to a paste composition, a biodegradable injectable paste obtained therefrom, and a method for producing the same, and more particularly, to a paste composition, which may offer the convenience of being applicable to the application site using one syringe by providing a crosslinked hydrogel as a paste phase, as well as biocompatibility, tackiness, and the balance between viscosity and elasticity, a biodegradable injectable paste obtained therefrom, and a method for producing the same.

### Background Art

Hydrogel is a three-dimensional structure composed of a network of hydrophilic molecules, and most of the components thereof are made of water. Hydrogels have high water content, porous structure, and biocompatibility properties.

Hydrogels can exhibit various properties depending on which polymer is selected as the main chain or which crosslinking method is used.

When synthetic compounds such as polyacrylic acid-based polymers or polyvinyl alcohol are used as the main chains, the hydrogels have low biocompatibility, but they are easy to chemically modify, making engineering applications thereof very easy. On the other hand, when natural compounds, especially collagen, fibrin, and hyaluronic acid, which are components of the extracellular matrix (ECM), are used as the main chains, the hydrogels are not easy to deform and thus engineering manipulation thereof is difficult, but have an advantage in that, since they are composed of bio-derived components, they have less side effects such immune and inflammatory responses when transplanted, indicating that they are suitable for clinical application.

Hyaluronic acid has a structure represented by Formula 1 below and can form hydrogels with various physical properties by covalent bonding:

[Formula 1]

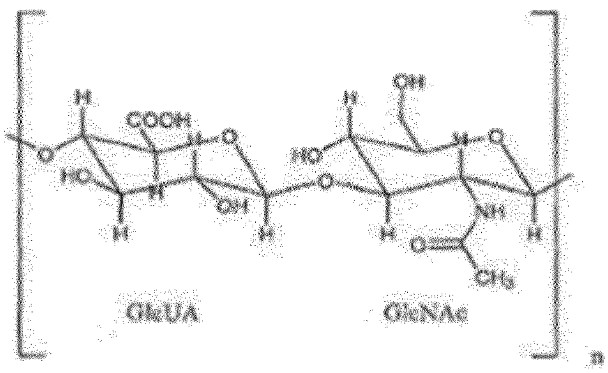

Gelatin is a natural polymer that is obtained by denaturing the triple-helix structure of collagen into a single-helix structure. Gelatin is classified into two types based on the processing method: gelatin A and gelatin B. Gelatin A may be produced by acid treatment, and gelatin B may be produced by base treatment.

Gelatin is widely used in tissue engineering due to its biocompatibility and ease of processing. In particular, gelatin hydrogels have many advantages in tissue regeneration because they offer easy delivery of growth factors, but gelatin hydrogels also have the problem of weak mechanical strength.

As described above, hydrogels have high biocompatibility due to their high water content and physicochemical similarity to the extracellular matrix, and thus various investigations have been made on the medical and pharmacological applications of hydrogels.

However, hydrogels have problems in that they have weak mechanical properties, and in particular, their low tensile strength limits their application in areas that must withstand load, and as a result, they are quickly lost through an early dissolution process at the target site.

As a related prior document, Korean Patent Application Publication No. 2017-7032839 discloses an attempt to offer viscoelastic properties while improving mechanical properties through PEGylation by multifunctional reactive

groups, on a linear or branched, multi-armed poly(ethylene glycol) (PEG).

However, in this case, there is a problem in that freezing, drying, grinding, and rehydration need to be performed for conversion to microgel particles that can offer a balance between viscosity and elasticity. Thus, there is still a need for research and development on a biodegradable injectable paste that is soluble and at the same time, can offer biocompatibility, tackiness, and a balance between viscosity and elasticity.

Furthermore, injectable hydrogels are crosslinked three-dimensional polymers and contain a significant amount of water, and thus they are widely used due to their excellent biocompatibility and biodegradability.

For example, when an injectable hydrogel (dry powder type, etc.) is used as a drug scaffold, two syringes are used to mix the injectable hydrogel with a drug solution and apply the mixture to the surgical site.

## DISCLOSURE

### Technical Problem

An object of the present invention is to provide a paste composition that may offer the convenience of being applied to the application site such as a surgical site using a crosslinked hydrogel and one syringe without the need to mix a drug solution, as well as biocompatibility, tackiness, and a balance between viscosity and elasticity.

Another object of the present invention is to provide a biodegradable injectable paste obtained from the paste composition and a method for producing the same.

The above and other objects of the present invention may all be achieved by the present invention described below.

### Technical Solution

To achieve the above objects,

the present invention provides a paste composition for controlled modification (control of water content) of a crosslinked product between polymers having biodegradable units and a monomer, the composition comprising at least two backbone polymers, a diglycidyl ether monomer, an active ingredient, and a carrier,

wherein the at least two backbone polymers, the diglycidyl ether monomer, the active ingredient, or the mixture thereof is uniformly distributed in the carrier, the backbone polymers or the active ingredient includes an adhesive polymer having a single-helix structure to form a scaffold backbone, and the carrier is not reactive with the backbone polymers, the diglycidyl ether monomer, and the active ingredient.

The carrier may be comprised in an amount of 80 wt% or more based on the total weight of the at least two backbone polymers, the adhesive polymer having the single-helix structure, the diglycidyl ether monomer, the active ingredient, and the carrier.

The adhesive polymer having the single-helix structure may be obtained by denaturing the triple-helix structure of collagen into a single-helix structure.

The adhesive polymer having the single-helix structure may be a polymer that is soluble under basic conditions, or a polymer that is soluble under acidic conditions.

When the adhesive polymer having the single-helix structure is included in a first backbone polymer among the at least two backbone polymers, the scaffold backbone may be formed by bonding between the adhesive polymer having the single-helix structure and the first backbone polymer.

When the adhesive polymer having the single-helix structure is included in the active ingredient, the scaffold backbone may be formed by bonding between the first backbone polymer and the second backbone polymer.

The first backbone polymer may be a hyaluronic acid-based or natural polymer selected from the group consisting of hyaluronic acid, hyaluronic acid salts, collagen, gelatin, chitosan, starch, alginic acid, and alginic acid salts.

The second backbone polymer may be a cellulose-based or synthetic polymer selected from the group consisting of carboxymethylcellulose, cellulose, polyvinylpyrrolidone, poloxamer, polyvinyl alcohol, and polyethylene glycol, and excludes types that overlap with the first backbone polymer.

The active ingredient included in the first backbone polymer or the second backbone polymer may be at least one selected from among starch, collagen, chitosan, carboxymethylcellulose, sodium hyaluronate, polyvinylpyrrolidone, poloxamer, polyethylene glycol, thrombin, fibrin, fibrinogen, tannic acid, vitamin K, bovine serum albumin (BSA), gentamicin, hemostatic agents, calcium chloride, drug carriers, wound dressings, skin soothing agents, moisturizing agents, anti-inflammatory agents, anti-atopic agents, and natural product active ingredients, and excludes types that overlap with the first or second backbone polymer.

The carrier may be provided as phosphate-buffered saline, purified water, water for injection, or saline.

In the paste composition, the weight ratio of the adhesive polymer having the single-helix structure: the at least two backbone polymers (as total weight): the diglycidyl ether monomer: the carrier: the active ingredient may be 0.1 to 15: 0.05 to 7: 0.00001 to 5: 82 to 95: 0 to 0.9.

The present invention also provides a biodegradable injectable paste obtained from the above-described paste composition, the biodegradable injectable paste comprising at least two backbone polymers, a diglycidyl ether monomer, an active ingredient, and a carrier, wherein the biodegradable injectable paste offers a form of micro-hydrogel particles that maintains a balance between viscosity and elasticity by including an adhesive polymer having a single-helix structure in the backbone polymers or the active ingredient, and the biodegradable injectable paste exhibits a paste form that is applicable to an application site using one syringe by comprising the carrier in an amount of 80 wt% based on the total weight of the backbone polymers, the diglycidyl ether monomer, the active ingredient, and the carrier.

The size of the micro-hydrogel particles may be 100 to 600 μm.

The biodegradable injectable paste may comprise undried powder having an average particle diameter of 50 to 400 μm, and may have a pushing force (injection or extrusion force) of 40 N or less, preferably 30 N, as measured using a universal tensile tester after filling a 10 ml syringe with 1 to 7 ml of the undried powder.

The biodegradable injectable paste may comprise adhesive polymer powder, and may have a tack force of 5 N or more, preferably 9 N or more, as measured according to a tack test using a rheometer.

In addition, the biodegradable injectable paste may have a water content of 80 to 95% as measured using a water content meter after applying 10 ml of water to 0.2 g of a sample, followed by standing at 37°C and 50 rpm for a predetermined time.

The biodegradable injectable paste was measured for blood absorption and time in addition to water absorption. An appropriate amount of blood was dropped onto a sample, and blood absorption and time were measured while the sample was turned at an angle of 180°every 10 seconds. Then, to determine the blood coagulation index (BCI), the absorbance was measured using a microplate reader. The biodegradable injectable paste may have a blood coagulation index (BCI) of 70% or more, preferably 74% or more, as calculated using Equation 1 below.

[Equation 1]

$$BCI\ (\%) = 100 - ((D_o/D_s) \times 100)$$

wherein $D_s$ denotes the absorbance of hemoglobin solution, and $D_o$ denotes the absorbance of hemolyzed whole blood. The BCI value means the blood coagulation rate, and a higher BCI value indicates better coagulation performance.

The biodegradable injectable paste may have a complex viscosity of 3,000,000 cP or more, preferably 10,000,000 cP or more, as measured using a rheometer at 0.1 Hz under the conditions of spindle P25 and 0.5 g of sample.

The biodegradable injectable paste was subjected to an *in vitro* degradation test using degrading enzymes, and the degree of degradation was checked overtime at 100 rpm in a 37°C shaking incubator. It was confirmed that almost all of the biodegradable injectable paste was degraded at 48 hours.

The present invention also provides a method for producing a biodegradable injectable paste, comprising:

a first step of preparing a base formulation by preparing a first backbone polymer and adding the same to a basic substance; a second step of preparing a porous scaffold by adding the base formulation to a diglycidyl ether monomer, followed by crosslinking; and a third step of purifying and grinding the porous scaffold to obtain a biodegradable injectable paste and binding an active ingredient to the surface of the biodegradable injectable paste,
wherein an adhesive polymer having a single-helix structure, which is soluble under basic conditions, is included in the base formulation or the biodegradable injectable paste, and the biodegradable injectable paste is provided in the form of a wet paste by a carrier provided during purification.

The basic substance that is used in the first step may be 0.1 to 1N sodium, potassium, or calcium hydroxide, preferably 0.1 to 0.5N sodium hydroxide.

The active ingredient included in the first backbone polymer or the second backbone polymer is preferably at least one selected from among starch, collagen, chitosan, carboxymethylcellulose, sodium hyaluronate, polyvinylpyrrolidone, poloxamer, polyethylene glycol, thrombin, fibrin, fibrinogen, tannic acid, vitamin K, bovine serum albumin (BSA), gentamicin, hemostatic agents, calcium chloride, drug carriers, wound dressings, skin soothing agents, moisturizing agents, anti-inflammatory agents, anti-atopic agents, and natural product active ingredients, and excludes types that overlap with the first or second backbone polymer.

The carrier is preferably provided as phosphate-buffered saline, purified water, water for injection, or saline.

Preferably, at least two backbone polymers, a diglycidyl ether monomer, the active ingredient, or the mixture thereof is uniformly distributed in the carrier, the backbone polymers or the active ingredient includes the adhesive polymer having the single-helix structure to form a scaffold backbone, and the carrier is not reactive with the backbone polymers,

the diglycidyl ether monomer, and the active ingredient.

In the biodegradable injectable paste, the weight ratio of the adhesive polymer having the single-helix structure: the at least two backbone polymers (as total weight): the diglycidyl ether monomer: the carrier: the active ingredient is preferably 0.1 to 15: 0.05 to 7: 0.00001 to 5: 82 to 95: 0 to 0.9.

The carrier is preferably comprised in an amount of 80 wt% or more based on the total weight of the at least two backbone polymers, the adhesive polymer having the single-helix structure, the diglycidyl ether monomer, the active ingredient, and the carrier.

The adhesive polymer having the single-helix structure is preferably obtained by denaturing the triple-helix structure of collagen into a single-helix structure.

When the adhesive polymer having the single-helix structure is included in the first backbone polymer among the at least two backbone polymers, the scaffold backbone is preferably formed by bonding between the adhesive polymer having the single-helix structure and the first backbone polymer.

When the adhesive polymer having the single-helix structure is included in the active ingredient, the scaffold backbone is preferably formed by bonding between the first backbone polymer and the second backbone polymer.

The third step may comprise neutralizing the porous scaffold while removing a residual crosslinking agent from the porous scaffold by up to 9 repeated purifications using a carrier of phosphate-buffered saline, purified water, water for injection, or saline, and then grinding the porous scaffold to have an average particle size ranging from 50 to 500 $\mu$m.

### Advantageous Effects

The biodegradable injectable paste obtained from the paste composition according to the present invention is soluble and has the effect of offering biocompatibility, tackiness, and a balance between viscosity and elasticity.

In particular, the biodegradable injectable paste according to the present invention comprises soluble wet powder uniformly distributed in a carrier, and thus may be used in applications, including hemostatic agents, bone fillers, wound dressings, drug carriers, tissue repair materials, skin soothing agents, moisturizing agents, anti-inflammatory agents, and anti-atopic agents.

### Brief Description of Drawings

FIG. 1 shows the appearance of a product according to Example 1 of the present invention. The micrograph on the right shows the product with a radius of 1 mm. In the right micrograph, black spots represent bubbles.

FIG. 2 shows photographs of the product according to Example 1 of the present invention after a blood absorption test. The micrograph on the right also shows the product with a radius of 1 mm. As can be seen in the microscope on the right, the blood has been well absorbed into the paste.

FIG. 3 is an SEM photograph taken drying the product according to Example 1 of the present invention. As can be seen in the photograph, porous beads can be seen.

### Mode for Invention

Hereinafter, the paste composition, biodegradable injectable paste, and production method therefor according to the present invention will be described in detail.

The present inventors have conducted studies on a biodegradable injectable paste which is applicable to the application site using one syringe without the need to use two syringes to mix a drug solution and apply the mixture to the application site, is soluble, and has biocompatibility, tackiness, and a balance between viscosity and elasticity. As a result, the present inventors have found that, when a paste composition provided in the form of a wet paste comprising a natural polymer having a specific structure in the backbone or on the surface and comprising undried powder obtained by crosslinking with a specific crosslinking agent and purification is used, all of the above-described objects can be achieved. The present inventors devoted themselves to research based on this finding, thereby completing the present invention.

### Paste Composition

In one embodiment of the present invention, the paste composition refers to a composition for controlled modification (control of water content) of a crosslinked product between polymers having biodegradable units and a monomer.

The paste composition comprises at least two backbone polymers, a diglycidyl ether monomer, an active ingredient, and a carrier, wherein the at least two backbone polymers, the diglycidyl ether monomer, the active ingredient,

or the mixture thereof is uniformly distributed in the carrier, and the backbone polymers or the active ingredient includes an adhesive polymer having a single-helix structure to form a scaffold backbone.

The carrier may not be reactive with the backbone polymers, the diglycidyl ether monomer, and the active ingredient. In this case, it is possible to provide a biodegradable injectable paste which is applicable to the application site using one syringe without the need to use two syringes to mix a drug solution and apply the mixture to the application site, is soluble, and has biocompatibility, tackiness, and a balance between viscosity and elasticity.

In one embodiment of the present invention, the paste composition includes wettability as one of the most important features.

Unless otherwise specified, the term "wettability" refers to a state in which the paste composition has an excessively water content of 80% or more as measured using a water content meter.

To achieve the above-described wettability, in one embodiment of the present invention, the carrier may be comprised in an amount of 80 wt% or more, specifically 82 to 95 wt%, based on the total weight of the at least two backbone polymers, the adhesive polymer having the single-helix structure, the diglycidyl ether monomer, the active ingredient, and the carrier. Within this range, the degradation rate of the paste composition is not reduced while the paste composition has an excessive water content, and the paste composition can be maintained stably until application.

The adhesive polymer having the single-helix structure may be obtained by denaturing the triple-helix structure of collagen into a single-helix structure.

Although the adhesive polymer having the single-helix structure may be a polymer that is soluble under acidic conditions, it is preferably a polymer that is soluble under basic conditions because it is easy to control the reaction thereof.

When the adhesive polymer having the single-helix structure is included in a first backbone polymer among the at least two backbone polymers, the scaffold backbone may be formed by bonding between the adhesive polymer having the single-helix structure and the first backbone polymer.

When the adhesive polymer having the single-helix structure is included in the active ingredient, the scaffold backbone may be formed by bonding between the first backbone polymer and the second backbone polymer.

The first backbone polymer may be a hyaluronic acid-based or natural polymer selected from the group consisting of hyaluronic acid, hyaluronic acid salts, collagen, gelatin, chitosan, starch, alginic acid, and alginic acid salts.

The second backbone polymer may be a cellulose-based or synthetic polymer selected from the group consisting of carboxymethylcellulose, cellulose, polyvinylpyrrolidone, poloxamer, polyvinyl alcohol, and polyethylene glycol, and excludes types that overlap with the first backbone polymer.

The diglycidyl ether monomer may be an alkanediol diglycidyl ether having 1 to 8 carbon atoms. In this case, crosslinking between the backbone polymers may be performed efficiently without deteriorating the mechanical properties of the backbone polymers.

The active ingredient may be, for example, at least one selected from among starch, collagen, chitosan, carboxymethylcellulose, sodium hyaluronate, polyvinylpyrrolidone, poloxamer, polyethylene glycol, thrombin, fibrin, fibrinogen, tannic acid, vitamin K, bovine serum albumin (BSA), gentamicin, hemostatic agents, calcium chloride, drug carriers, wound dressings, skin soothing agents, moisturizing agents, anti-inflammatory agents, anti-atopic agents, and natural product active ingredients.

The carrier may be provided as phosphate-buffered saline, purified water, water for injection, or saline. As described above, the carrier is preferably comprised in an amount of 80 wt% or more, specifically 82 to 95 wt%, based on the total weight of the backbone polymers, the diglycidyl ether monomer, the active ingredient, and water. In this case, the paste composition exhibits excessive wettability.

In the paste composition, the weight ratio of the adhesive polymer having the single-helix structure: the backbone polymers (polymers excluding the adhesive polymer having the single-helix structure, which is included in the backbone polymers): the diglycidyl ether monomer: water: the active ingredient is, for example, 0.1 to 15: 0.05 to 7: 0.00001 to 5: 82 to 95: 0 to 0.9. In this case, the paste composition is soluble and has excellent biocompatibility, tackiness, and balance between viscosity and elasticity.

In the paste composition, the weight ratio of the adhesive polymer having the single-helix structure: the at least two backbone polymers: the diglycidyl ether monomer: water: the active ingredient is, for example, 0.1 to 15: 0.05 to 7: 0.00001 to 5: 82 to 95: 0 to 0.9, specifically 0.15 to 14: 0.3 to 6.6: 0.00001 to 5: 84 to 93: 0.1-0.35. In this case, the paste composition is soluble and has excellent biocompatibility, tackiness, and balance between viscosity and elasticity.

**<Biodegradable Injectable Paste>**

In one embodiment of the present invention, the biodegradable injectable paste is preferably obtained from the above-described paste composition for controlled modification (control of water content) of the crosslinked product between polymers having biodegradable units and a monomer.

The biodegradable injectable paste comprises, for example, at least two backbone polymers, a diglycidyl ether monomer, an active ingredient, and a carrier. The biodegradable injectable paste offers a form of micro-hydrogel particles

that maintains a balance between viscosity and elasticity by including an adhesive polymer having a single-helix structure in the backbone polymers or the active ingredient, and the biodegradable injectable paste exhibits a paste form that is applicable to an application site using one syringe by comprising the carrier in an amount of 80 wt% based on the total weight of the backbone polymers, the diglycidyl ether monomer, the active ingredient, and the carrier. In this case, the biodegradable injectable paste is soluble and has excellent biocompatibility, tackiness, and balance between viscosity and elasticity.

The size of the micro-hydrogel particles may be, for example, 100 to 600 μm, specifically 200 to 400 um. In this case, the biodegradable injectable paste may exhibit the optimal maximum effect in terms of water absorption rate.

The biodegradable injectable paste comprises undried powder with an average particle diameter of 50 to 400 um, specifically 100 to 400 um, preferably 200 to 400 μm, and is not subjected to drying treatment for the purpose of providing excessive wettability.

When the biodegradable injectable paste has the average particle size within the above-described range, it is easy to produce and has excellent blood absorption ability. The average particle size may be measured by SEM image analysis.

The biodegradable injectable paste may have a syringe pushing force (injection or extrusion force) that is 5 times or less, specifically 1 to 4 times, the tack force. In this case, the biodegradable injectable paste is easy to use and sticks well to the application site, thereby exhibiting increased efficacy.

The injection force may be measured using a 10 ml syringe and a universal tensile tester.

The tack force may be measured using a rheometer.

The biodegradable injectable paste was measured for blood absorption and time by dropping blood onto the paste sample and turning the sample at an angle of 180° immediately after standing for time a. Also, the paste was measured for water absorption by absorbing water into the paste according to a water absorption test. To determine the blood coagulation index (BCI), the absorbance was measured using a microplate reader. The biodegradable injectable paste may have a blood coagulation index (BCI) of 70% or more, preferably 74% or more, as calculated using Equation 1 below.

[Equation 1]

$$BCI\ (\%) = 100 - ((D_o/D_s)\ X\ 100)$$

wherein $D_s$ denotes the absorbance of hemoglobin solution, and $D_o$ denotes the absorbance of hemolyzed whole blood. The BCI value means the blood coagulation rate, and a higher BCI value indicates better coagulation performance. After 0.2 ml of blood was applied to 0.2 g of the sample, the sample was turned at an angle of 180° at 10-second intervals and the time taken for the blood to be completely absorbed was measured. The water absorption ability was evaluated by applying 10 ml of water to 0.2 g of the sample, standing at 37°C and 50 rpm for 24 hours, and then measuring the weight.

The biodegradable injectable paste may preferably have a water content of 80% or more, specifically 80 to 95%, as measured using a water content meter. In this case, the biodegradable injectable paste is applicable to an application site such as a surgical site using one syringe without the need to mix a drug solution using two syringes.

The biodegradable injectable paste may have a complex viscosity of 3,000,000 cP or more, preferably 9,000,000 cP or more, as measured using a rheometer at 0.1 Hz under the conditions of spindle P25 and 0.5 g of sample.

The biodegradable injectable paste may exhibit a cell viability of 80% or more, preferably 89% or more, as measured according to the ISO 10993 cytotoxicity measurement method.

The biodegradable injectable paste has no detected bacteria as a result of measurement according to the Korean Pharmacopoeia sterility test method.

The biodegradable injectable paste has no cytotoxicity as a result of measurement according to a cytotoxicity test method.

The biodegradable injectable paste has a residual amount of crosslinking agent of 1 wt% or less as a result of measuring the residual amount of crosslinking agent according to an internal standard method using GC chromatography system.

**<Method for Producing Biodegradable Injectable Paste>**

A method for producing a biodegradable injectable paste according to one embodiment comprises: a first step of preparing a base formulation; a second step of preparing a scaffold; and a third step of obtaining a biodegradable injectable paste.

Specifically, the first step may comprise preparing a base formulation by preparing a first backbone polymer and adding the same to a basic substance.

The first backbone polymer may include an adhesive polymer having a single-helix structure, which is soluble

under basic conditions. In this case, it is possible to produce a paste having an appropriate water content, which may continuously maintain its shape.

When an adhesive polymer having a single-helix structure, which is soluble under basic conditions, is included as the backbone polymer, an additional polymer corresponding to the remaining backbone polymer may be a hyaluronic acid-based or natural polymer selected from the group consisting of hyaluronic acid, hyaluronic acid salts, collagen, gelatin, chitosan, starch, alginic acid, and alginic aid salts. In this case, it is possible to provide a synergy effect between extrusion force and tack force.

When the first backbone polymer does not include an adhesive polymer having a single-helix structure, which is soluble under basic conditions, the at least two backbone polymers may include the above-described first backbone polymer and a second separate backbone polymer different therefrom. Here, the second backbone polymer may be a cellulose-based or synthetic polymer selected from the group consisting of carboxymethylcellulose, cellulose, polyvinylpyrrolidone, poloxamer, polyvinyl alcohol, and polyethylene glycol, and excludes types that overlap with the first backbone polymer.

Furthermore, the first backbone polymer and the second backbone polymer may include an active ingredient as needed. The active ingredient included in the first backbone polymer or the second backbone polymer is preferably at least one selected from among starch, collagen, chitosan, carboxymethylcellulose, sodium hyaluronate, polyvinylpyrrolidone, poloxamer, polyethylene glycol, thrombin, fibrin, fibrinogen, tannic acid, vitamin K, bovine serum albumin (BSA), gentamicin, hemostatic agents, calcium chloride, drug carriers, wound dressings, skin soothing agents, moisturizing agents, anti-inflammatory agents, anti-atopic agents, and natural product active ingredients, and excludes types that overlap with the first or second backbone polymer.

The basic substance that is used in the first step may preferably include 0.1 to 0.5N alkali metal such as sodium, potassium, or calcium hydroxide. In this case, it is possible to increase the solubility of the base formulation.

The second step may comprise preparing a porous scaffold by adding the base formulation to a diglycidyl ether monomer, followed by crosslinking

The third step may comprise purifying and grinding the porous scaffold to obtain a biodegradable injectable paste and binding an active ingredient to the surface of the biodegradable injectable paste.

An adhesive polymer having a single-helix structure, which is soluble under basic conditions, may be included in the biodegradable injectable paste.

The third step may comprise neutralizing the porous scaffold while removing the remaining crosslinking agent from the porous scaffold by up to 9 repeated purifications using phosphate-buffered saline, purified water, water for injection, or saline, and then grinding the porous scaffold to an average particle size ranging from 50 to 500 μm.

The biodegradable injectable paste may be provided in the form of a wet paste by the water provided during the purification.

In particular, when the biodegradable injectable paste of the present invention is used as a hemostatic agent, it may exhibit excellent performance as a hemostatic agent due to the improved hemostatic ability of the active ingredient.

In particular, when the biodegradable injectable paste of the present invention is used as a bone filler, it may exhibit excellent performance as a bone filler due to the improved physical properties of the active ingredient.

That is, the biodegradable injectable paste according to embodiments of the present invention comprises soluble wet powder uniformly distributed in the carrier, and thus may be used in applications, including hemostatic agents, bone fillers, wound dressings, drug carriers, tissue repair materials, skin soothing agents, moisturizing agents, anti-inflammatory agents, and anti-atopic agents.

Hereinafter, preferred examples will be described to aid understanding of the present invention. However, the following examples are only illustrative of the present invention, and it will be obvious to those skilled in the art that various changes and modifications are possible without departing from the scope and technical spirit of the present invention. It is to be understood that such changes and modifications fall within the scope of the appended claims.

**[Examples: Production of Biodegradable Injectable Pastes]**

**Example 1**

For the production of a paste composition of Example 1, gelatin and sodium hyaluronate were prepared.

Specifically, 30 wt% of gelatin and 0.84 wt% of sodium hyaluronate were mixed and dissolved in 0.25N NaOH.

0.84 wt% of 1,4-butanediol diglycidyl ether was added to the solution, followed by crosslinking at 30°C for 18 hours, thereby preparing a support backbone.

The crosslinked product was purified 8 or 9 times repeatedly using phosphate-buffered saline to remove residual butanediol diglycidyl ether and neutralize to a neutral pH.

The obtained biodegradable injectable paste was composed of 13.64 wt% of gelatin, 0.38 wt% of sodium hyaluronate, 0.0002 wt% of 1.4-butanediol diglycidyl ether, and 85.84 wt% of phosphate-buffered saline.

The phosphate-buffered saline used was a mixture of 84.87 wt% of purified water, 0.71 wt% of sodium chloride, 0.04 wt% of potassium dihydrogen phosphate, and 0.22 wt% of sodium dibasic phosphate dodecahydrate.

Then, the biodegradable injectable paste was ground using a plunger mill to have an average particle diameter of 300 μm.

Then, the powder was mixed with 0.14 wt% of calcium chloride dihydrate, thereby producing a biodegradable injectable paste in the form of a paste having the active ingredient bound to the surface.

## Example 2

For the production of a paste composition of Example 2, sodium hyaluronate and carboxymethyl cellulose were prepared.

Specifically, 15.24 wt% of sodium hyaluronate and 7.62 wt% of carboxymethyl cellulose were mixed and dissolved in 25N NaOH.

0.95 wt% of 1,4-butanediol diglycidyl ether was added to the solution, followed by crosslinking at 30°C for 18 hours, thereby preparing a scaffold backbone.

The crosslinked product was purified 8 or 9 times repeatedly using phosphate-buffered saline to remove the remaining butanediol diglycidyl ether and neutralize to a neutral pH.

The biodegradable injectable paste obtained after purification was composed of 4.32 wt% of sodium hyaluronate, 2.16 wt% of carboxymethyl cellulose, 0.0002 wt% of 1,4-butanediol diglycidyl ether, and 93.18 wt% of phosphate-buffered saline.

Then, the biodegradable injectable paste was ground using a plunger mill to have an average particle diameter of 300 μm.

Then, the powder was mixed with 0.14 wt% of calcium chloride dihydrate and 0.2 wt% of gelatin, thereby producing a biodegradable injectable paste in the form of a paste having the active ingredient bound to the surface.

## Example 3

A biodegradable injectable paste was produced in the form of a paste by repeating the same process as Example 1, except that calcium chloride dihydrate was not used while gelatin in an amount equal to the calcium chloride dihydrate content used in Example 1 was further used.

## Example 4

A biodegradable injectable paste was produced in the form of a paste by repeating the same process as Example 2, except that calcium chloride dihydrate was not used while gelatin in an amount equal to the calcium chloride dihydrate content used in Example 2 was further used.

## Comparative Example 1

The present inventors sought to produce a biodegradable injectable paste in the form of a paste by repeating the same process as Example 1, except that gelatin was not used while sodium hyaluronate in an amount equal to the gelatin content used in Example 1 was further used. However, the product was in the form of beads and it was impossible to produce a paste.

## Comparative Example 2

A biodegradable injectable paste was produced in the form of a paste by repeating the same process as Example 1, except that sodium hyaluronate was not used while gelatin in an amount equal to the sodium hyaluronate content used in Example 1 was further used.

## Comparative Example 3

A biodegradable injectable paste was prepared in the form of a paste by repeating the same process as Example 1, except that 10 wt% of gelatin was used.

## Comparative Example 4

A biodegradable injectable paste was prepared in paste form by repeating the same process as Example 2,

except that sodium hyaluronate was excluded and only carboxymethyl cellulose was used.

**Comparative Example 5**

Crosslinked hyaluronic acid was produced in the form of a paste by repeating the same process as Example 1, except that polyethylene glycol diacrylate (PEGDA) was used instead of 1,4-butanediol diglycidyl ether.

**Comparative Example 6**

A biodegradable injectable paste was prepared in the form of a paste by repeating the same process as Example 1, except that freeze drying was performed after purification and grinding.

**Reference Example 1**

A biodegradable injectable paste was prepared in the form of a paste by repeating the same process as Example 1, except that the average particle size of 300 um in Example 1 was changed to 700 μm.

**Reference Example 2**

The present inventors sought to produce a biodegradable injectable paste in the form of a paste by repeating the same process as Example 1, except that the average particle size of 300 um in Example 1 was changed to 30 um. However, it was impossible to produce the sample because the mesh size of the grinding mill was excessively small.

**Test Example**

For the pastes produced in Examples 1 to 4, Comparative Examples 2 to 6, and Reference Example 1, tests for measuring complex viscosity, tack force, injection force, hemostatic performance (blood absorption ability), and blood coagulation index (BCI), and cytotoxicity and sterility tests were performed as described below, and the measurement results are shown in Table 1 below.

**<Methods for Measuring Physical Properties>**

*Complex viscosity: According to the complex viscosity measurement method, the complex viscosity at 0.1 Hz was measured using a rheometer under the conditions of spindle P25 and 0.5 g of sample.
*Tack force: According to the tack test method under the conditions of spindle P25 and 0.5 g of sample, the maximum force when the sample solution was raised at a speed of 1.25 mm/min after pressed with a constant force (1 N) was measured using a rheometer.
*Injection force: According to the injectability test, the injection force was measured using a universal material tester under the condition of speed of 1 mm/s.
*Hemostatic performance (blood absorption ability): Blood was dropped onto the sample. If the blood immediately fell from the sample, the blood absorption time was recorded as 0 seconds, and if it did not fall immediately, whether it fell was checked for up to 60 seconds while the sample was turned at an angle of 180° every 10 seconds.
*Blood coagulation index (BCI): The BCI was measured using a microplate reader according to the blood coagulation index test method.
*Cytotoxicity: The cytotoxicity was measured according to the cytotoxicity test method, and cell viability was expressed as %.
*Sterility test: The presence or absence of bacteria was evaluated according to the sterility test method and the result was expressed as presence or absence.
*Water content: The water content of the sample was measured using a water content meter.

[Table 1]

| | Complex viscosity (cP) | Tack force (N) | Injection force (N) | Blood absorption (sec) | BCI(%) | Cytotoxicity (%) | Sterility test (presence /absence) | Water content (%) |
|---|---|---|---|---|---|---|---|---|
| Example 1 | 11,782,000 | 11 | 8.98 | 60 | 80 | 111.25 | Absence | 85 |
| Example 2 | 11,963,333 | 10.34 | 8.65 | 60 | 78 | 92.25 | Absence | 85 |
| Example 3 | 12,850,000 | 9.28 | 8.89 | 60 | 75 | 89.50 | Absence | 85 |
| Example 4 | 11,790,000 | 9 | 8.20 | 60 | 74 | 100.00 | Absence | 85 |
| Comparative Example 2 | 7,086,000 | 3.84 | 7.78 | 40 | 75 | 88.50 | Absence | 84 |
| Comparative Example 3 | 3,114,000 | 2.796 | 20.01 | 20 | 69 | 85.50 | Absence | 80 |
| Comparative Example 4 | 11,990,000 | 5.59 | 9.77 | 30 | 70 | 89.00 | Absence | 50 |
| Comparative Example 5 | 4,945,000 | 6.4 | 9.69 | 20 | 65 | 30.00 | Absence | 60 |
| Comparative Example 6 | - | - | - | 60 | 79 | 87.00 | Absence | 80 |
| Reference Example 1 | 12,110,000 | 2.796 | 9.05 | 10 | 73 | 91.00 | Absence | 84 |

As shown in Table 1 above, Examples 1 to 4 corresponding to the biodegradable injectable paste of the present invention exhibited values within appropriate ranges for all measurement items, including complex viscosity, tack force, injection force, blood absorption ability, blood coagulation index (BCI), cytotoxicity, sterility test, and water content, indicating that they are soluble and offer biocompatibility, tackiness, and a balance between viscosity and elasticity.

On the other hand, in the case of Comparative Examples 1 to 6, which do not correspond to the biodegradable injectable paste of the present invention, it was confirmed that production was impossible, or even if production was possible, tack force, blood absorption, injection force, etc. would be inadequate.

Specifically, in the case of Comparative Example 1 in which the first backbone polymer was used in an amount equal to the adhesive polymer content and the adhesive polymer was not used, the paste could not be produced due to insufficient adhesive force.

In addition, in the case of Comparative Example 2 in which the adhesive polymer was used in an amount equal to the content of the first backbone polymer, it was confirmed that the tack force was poor.

In addition, in the case of Comparative Example 3 in which the adhesive polymer was added in an inappropriately excessive amount, and in the case of Comparative Example 4 in which the second backbone polymer was used alone, it was confirmed that the blood absorption ability was low.

In addition, Comparative Example 5 in which polyethylene glycol diacrylate different from that used in the Examples was used as the crosslinking agent, it was confirmed that cytotoxicity was high.

In addition, in the case of Comparative Example 6 in which the obtained paste form was processed into a dry powder type, it was confirmed that the extrusion force was relatively high, indicating that the processability was poor.

Furthermore, when the obtained paste was ground to an excessively large size, the blood absorption ability was poor, as shown in Reference Example 1, and when the obtained paste was ground to an excessively small size, production was impossible, as shown in Reference Example 2.

Therefore, it can be seen that the biodegradable injectable paste of the present invention, which is provided in the form of a wet paste comprising a natural polymer having a specific structure in the backbone or on the surface and obtained by crosslinking with a predetermined crosslinking agent and purification, is used, the biodegradable injectable paste is provided uniformly distributed in the carrier, and thus is suitable for use in applications, including hemostatic agents, bone fillers, wound dressings, drug carriers, tissue repair materials, skin soothing agents, moisturizing agents, anti-inflammatory agents, and anti-atopic agents.

## Claims

1. A paste composition for controlled modification (control of water content) of a crosslinked product between polymers having biodegradable units and a monomer, the composition comprising at least two backbone polymers, a diglycidyl ether monomer, an active ingredient, and a carrier,
wherein the at least two backbone polymers, the diglycidyl ether monomer, the active ingredient, or the mixture thereof is uniformly distributed in the carrier, the backbone polymers or the active ingredient includes an adhesive polymer having a single-helix structure to form a scaffold backbone, and the carrier is not reactive with the backbone polymers, the diglycidyl ether monomer, and the active ingredient.

2. The paste composition of claim 1, wherein the carrier is comprised in an amount of 80 wt% or more based on the total weight of the at least two backbone polymers, the adhesive polymer having the single-helix structure, the diglycidyl ether monomer, the active ingredient, and the carrier.

3. The paste composition of claim 1, wherein the adhesive polymer having the single-helix structure may be obtained by denaturing a triple-helix structure of collagen into a single-helix structure.

4. The paste composition of claim 1, wherein, when the adhesive polymer having the single-helix structure is included in a first backbone polymer among the at least two backbone polymers, the scaffold backbone is formed by bonding between the adhesive polymer having the single-helix structure and the first backbone polymer.

5. The paste composition of claim 1, wherein, when the adhesive polymer having the single-helix structure is included in the active ingredient, the scaffold backbone is formed by bonding between the first backbone polymer and the second backbone polymer.

6. The paste composition of claim 1, wherein the first backbone polymer is a hyaluronic acid-based or natural polymer selected from the group consisting of hyaluronic acid, hyaluronic acid salts, collagen, gelatin, chitosan, starch, alginic acid, and alginic acid salts.

7. The paste composition of claim 1, wherein the second backbone polymer is a cellulose-based or synthetic polymer selected from the group consisting of carboxymethylcellulose, cellulose, polyvinylpyrrolidone, poloxamer, polyvinyl alcohol, and polyethylene glycol, and excludes types that overlap with the first backbone polymer.

8. The paste composition of claim 1, wherein the active ingredient is at least one selected from among starch, collagen, chitosan, carboxymethylcellulose, sodium hyaluronate, polyvinylpyrrolidone, poloxamer, polyethylene glycol, thrombin, fibrin, fibrinogen, tannic acid, vitamin K, bovine serum albumin (BSA), gentamicin, hemostatic agents, calcium chloride, drug carriers, wound dressings, skin soothing agents, moisturizing agents, anti-inflammatory agents, anti-atopic agents, and natural product active ingredients, and excludes types that overlap with the first backbone polymer or the second backbone polymer.

9. The paste composition of claim 1, wherein the carrier is provided as phosphate-buffered saline, purified water, water for injection, or saline.

10. The paste composition of claim 1, wherein the weight ratio of the adhesive polymer having the single-helix structure: the at least two backbone polymers (as total weight): the diglycidyl ether monomer: the carrier: the active ingredient is 0.1 to 15: 0.05 to 7: 0.00001 to 5: 82 to 95: 0 to 0.9.

11. A biodegradable injectable paste obtained from the paste composition of claim 1, the biodegradable injectable paste comprising at least two backbone polymers, a diglycidyl ether monomer, an active ingredient, and a carrier,
wherein the biodegradable injectable paste offers a form of micro-hydrogel particles that maintains a balance between viscosity and elasticity by including an adhesive polymer having a single-helix structure in the backbone polymers or the active ingredient, and the biodegradable injectable paste exhibits a paste form that is applicable to an application site using one syringe by comprising the carrier in an amount of 80 wt% based on the total weight of the backbone polymers, the diglycidyl ether monomer, the active ingredient, and the carrier.

12. The biodegradable injectable paste of claim 11, wherein the micro-hydrogel particles have a size of 100 to 600 $\mu$m.

13. The biodegradable injectable paste of claim 11, wherein the biodegradable injectable paste comprises undried

powder having an average particle diameter of 50 to 400 um, and has a pushing force (injection or extrusion force) of 40 N or less as measured using a universal tensile tester after filling a 10 ml syringe with 1 to 7 ml of the undried powder.

**14.** The biodegradable injectable paste of claim 11, wherein the biodegradable injectable paste comprises adhesive polymer powder having a tack force of 5 N or more as measured according to a tack test using a rheometer.

**15.** The biodegradable injectable paste of claim 11, wherein the biodegradable injectable paste has a water content of 80 to 95% as measured using a water content meter after applying 10 ml of water to 0.2 g of a sample, followed by standing at 37°C and 50 rpm.

**16.** The biodegradable injectable paste of claim 11, wherein the biodegradable injectable paste has a blood coagulation index (BCI) of 70% or more as determined by dropping a predetermined amount of blood onto a sample, measuring blood absorption and time while turning the sample at an angle of 180° every 10 seconds, measuring absorbance using a microplate reader according to a blood coagulation index test method, and then calculating the blood coagulation index (BCI) using Equation 1 below:

[Equation 1]

$$BCI\ (\%) = 100 - ((D_o/D_s) \times 100)$$

wherein $D_s$ denotes the absorbance of hemoglobin solution, $D_o$ denotes the absorbance of hemolyzed whole blood, the BCI value means blood coagulation rate, and a higher BCI value indicates better coagulation performance.

**17.** The biodegradable injectable paste of claim 11, wherein the biodegradable injectable paste has a complex viscosity of 3,000,000 cP or more as measured at 0.1 Hz using a rheometer under conditions of spindle P25 and 0.5 g of sample.

**18.** A method for producing a biodegradable injectable paste, comprising:

a first step of preparing a base formulation by preparing a first backbone polymer and adding the same to a basic substance;
a second step of preparing a porous scaffold by adding the base formulation to a diglycidyl ether monomer, followed by crosslinking with a second backbone polymer; and
a third step of purifying and grinding the porous scaffold to obtain a biodegradable injectable paste and binding an active ingredient to a surface of the biodegradable injectable paste,
wherein an adhesive polymer having a single-helix structure, which is soluble under basic conditions, is included in the base formulation or the biodegradable injectable paste, and
the biodegradable injectable paste is provided in the form of a wet paste by a carrier provided during the purification.

FIG. 1

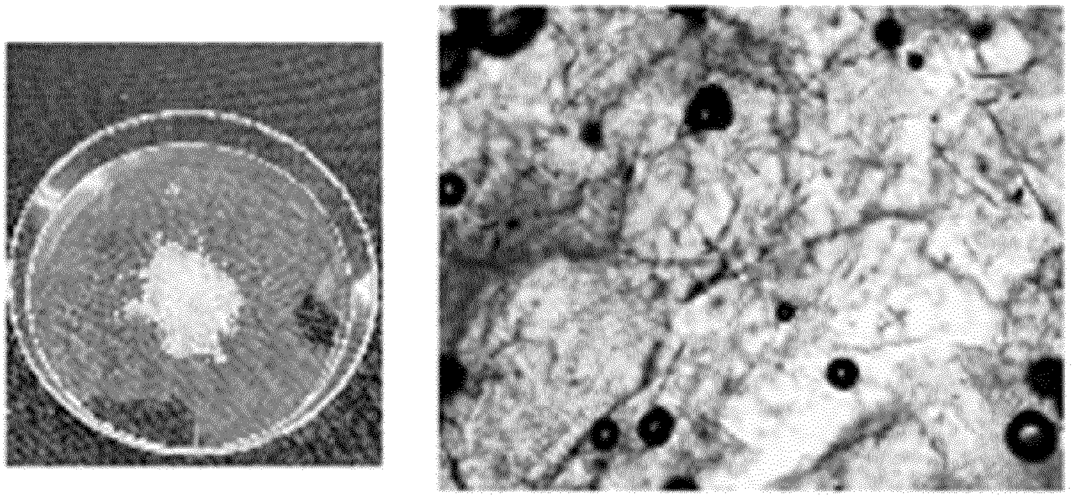

FIG. 2

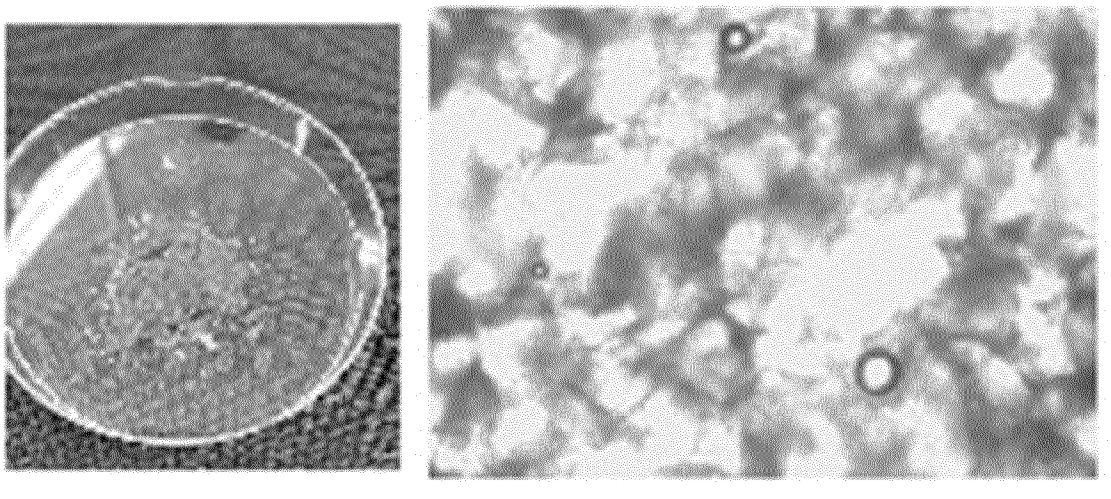

FIG. 3

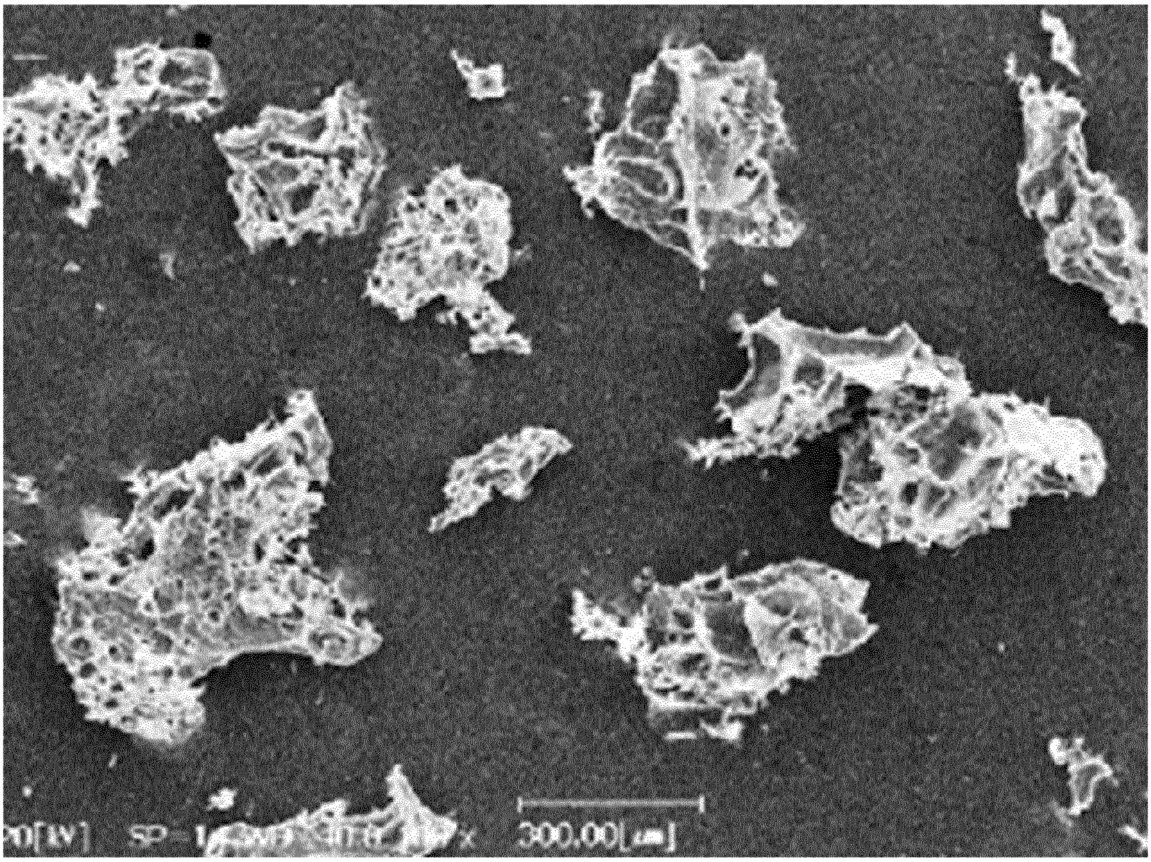

## INTERNATIONAL SEARCH REPORT

International application No.
**PCT/KR2023/003119**

### A. CLASSIFICATION OF SUBJECT MATTER

**C08G 83/00**(2006.01)i; **C08J 9/40**(2006.01)i; **C08L 5/08**(2006.01)i; **C08L 101/00**(2006.01)i; **A61L 24/08**(2006.01)i; **A61L 24/00**(2006.01)i; **A61K 9/00**(2006.01)i; **A61K 47/30**(2006.01)i

According to International Patent Classification (IPC) or to both national classification and IPC

### B. FIELDS SEARCHED

Minimum documentation searched (classification system followed by classification symbols)

C08G 83/00(2006.01); A61F 2/00(2006.01); A61K 31/728(2006.01); A61K 47/48(2006.01); A61K 8/02(2006.01); A61K 8/65(2006.01); A61K 8/73(2006.01); A61L 15/28(2006.01); A61L 27/52(2006.01); C08J 3/12(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 주사용 페이스트(injectable paste), 젤라틴(gelatin), 히알루론산(hyaluronic acid), 셀룰로오스(cellulose), 디글리시딜 에테르(diglycidyl ether), 활성 성분(active component), 생분해성(biodegradable)

### C. DOCUMENTS CONSIDERED TO BE RELEVANT

| Category* | Citation of document, with indication, where appropriate, of the relevant passages | Relevant to claim No. |
|---|---|---|
| X | KR 10-2007-0046093 A (FERROSAN A/S) 02 May 2007 (2007-05-02) See paragraphs [0036], [0042], [0060], [0077], [0078], [0106], [0107], [0156]-[0168], [0186] and [0241]. | 1-4,6,8-10 |
| Y | | 5,7,11-18 |
| Y | KR 10-2013-0123080 A (GENEWEL CO., LTD.) 12 November 2013 (2013-11-12) See claims 8, 9, 13 and 15. | 5,7,11-18 |
| A | KR 10-2021-0148945 A (LG CHEM, LTD.) 08 December 2021 (2021-12-08) See entire document. | 1-18 |
| A | US 2010-0316683 A1 (PIRON, Estelle et al.) 16 December 2010 (2010-12-16) See entire document. | 1-18 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 June 2023** | **07 June 2023** |

| Name and mailing address of the ISA/KR | Authorized officer |
|---|---|
| **Korean Intellectual Property Office Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2023/003119**

**C. DOCUMENTS CONSIDERED TO BE RELEVANT**

| Category* | Citation of document, with indication, where appropriate, of the relevant passages | Relevant to claim No. |
|---|---|---|
| A | KR 10-2094407 B1 (ULTRA V CO., LTD.) 31 March 2020 (2020-03-31)<br>See entire document. | 1-18 |

Form PCT/ISA/210 (second sheet) (July 2022)

**INTERNATIONAL SEARCH REPORT**
**Information on patent family members**

International application No.
**PCT/KR2023/003119**

| Patent document cited in search report | Publication date (day/month/year) | Patent family member(s) | Publication date (day/month/year) |
|---|---|---|---|
| KR 10-2007-0046093 A | 02 May 2007 | CA 2571981 A1 | 19 January 2006 |
| | | CA 2571981 C | 30 December 2014 |
| | | CN 101001649 A | 18 July 2007 |
| | | CN 101001649 B | 31 August 2011 |
| | | EP 1786480 A1 | 23 May 2007 |
| | | EP 1786480 B1 | 21 September 2016 |
| | | JP 2008-505132 A | 21 February 2008 |
| | | JP 4959557 B2 | 27 June 2012 |
| | | TW 200605921 A | 16 February 2006 |
| | | US 2007-0009578 A1 | 11 January 2007 |
| | | US 8021684 B2 | 20 September 2011 |
| | | WO 2006-005340 A1 | 19 January 2006 |
| KR 10-2013-0123080 A | 12 November 2013 | KR 10-1374271 B1 | 14 March 2014 |
| KR 10-2021-0148945 A | 08 December 2021 | CN 114269813 A | 01 April 2022 |
| | | EP 3995531 A1 | 11 May 2022 |
| | | JP 2022-544815 A | 21 October 2022 |
| | | US 2022-0331260 A1 | 20 October 2022 |
| | | WO 2021-246764 A1 | 09 December 2021 |
| US 2010-0316683 A1 | 16 December 2010 | CA 2671793 A1 | 12 June 2008 |
| | | CN 101594892 A | 02 December 2009 |
| | | EP 2152329 A1 | 17 February 2010 |
| | | EP 2152329 B1 | 15 February 2017 |
| | | EP 2489374 A1 | 22 August 2012 |
| | | ES 2632947 T3 | 18 September 2017 |
| | | FR 2909560 A1 | 13 June 2008 |
| | | JP 2010-511454 A | 15 April 2010 |
| | | JP 5642388 B2 | 17 December 2014 |
| | | KR 10-1642516 B1 | 25 July 2016 |
| | | KR 10-1642622 B1 | 25 July 2016 |
| | | KR 10-2009-0085102 A | 06 August 2009 |
| | | KR 10-2014-0101018 A | 18 August 2014 |
| | | WO 2008-068297 A1 | 12 June 2008 |
| | | ZA 200903919 B | 30 June 2010 |
| KR 10-2094407 B1 | 31 March 2020 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 20177032839